# EUROPÄISCHE PATENTSCHRIFT

(11) **EP 2 345 406 B1**
(45) Veröffentlichungstag und Bekanntmachung des Hinweises auf die Patenterteilung: **12.10.2016**
(21) Anmeldenummer: 10191623.7
(22) Anmeldetag: 18.11.2010
(51) Int. Cl.: A61K 9/00, A61K 47/10, A61K 31/047

(54) **Zusammensetzungen aus Dimerdiol als Körperflüssigkeitsersatzstoffe**
Use of dimer diol in the production of bodily fluid substitutes
Utilisation de dimerdiol pour la fabrication de produits de substitution de liquides corporels

(30) Priorität: 16.01.2010 EP 10000372
(43) Veröffentlichungstag der Anmeldung: 20.07.2011
(73) Patentinhaber: Aachener Centrum für Technologietransfer in der Ophthalmologie e.V., 52070 Aachen (DE)
(72) Erfinder: Schrage, Norbert, 52070 Aachen (DE)
(74) Vertreter: Cabinet Plasseraud

(56) Entgegenhaltungen:
- EP-B1- 1 471 898
- DE-A1- 19 507 203
- US-A- 5 811 446
- US-A1- 2001 018 424
- Bodige, S.G: "Structure of an unusual cage dimer diol", Journal of chemical Crystallography, Bd. 29, Nr. 12 1. Dezember 1999 (1999-12-01), Seiten 1261-1263, XP002619721, DOI: 10.1023/A:1009569319307 Gefunden im Internet: URL:http://www.springerlink.com/content/kh 608n1l333ux840/fulltext.pdf [gefunden am 2011-02-01]
- JOHANNES NEPP ET AL: 'The clinical use of viscoelastic artificial tears and sodium chloride in dry-eye syndrome' BIOMATERIALS Bd. 22, Nr. 24, 01 Dezember 2001, Seiten 3305 - 3310, XP055156920 DOI: 10.1016/S0142-9612(01)00167-3 ISSN: 0142-9612

## Beschreibung

Die gegenwärtige Erfindung betrifft Zusammensetzungen enthaltend mindestens 90 Gewichtprozent Dimerdiol zur Anwendung in einem Verfahren zur chirurgischen oder therapeutischen Behandlung des menschlichen oder tierischen Körpers.

Der Ersatz von Körperflüssigkeiten im Bereich von Schleimhäuten und inneren Körperhöhlen welche mit Schleimhaut oder Epithelzellen ausgekleidet sind (außer Blutgefäßen) ist derzeit eine typische Anwendung für wässrige Flüssigkeiten wie z.B. Tränenersatzstoffe bei den Tränenwegen oder künstlicher Speichel im Bereich der Mundhöhle, aber auch für Silikonöl oder Fluorcarbonflüssigkeiten, die als Endotamponaden im Bereich des Glaskörpers des Auges verwendet werden (wie zum Beispiel in US 5,258,412 beschrieben).

Die Besonderheit der menschlichen oder tierischen Körperoberfläche ist, dass wässrige Flüssigkeiten dort in der Regel einem starken Stoffaustausch unterliegen und damit meist unerwünscht kurz auf der Körperoberfläche verbleiben. Dahingegen sind Öle, wie z.B. Silikonöl, in der Regel als lang verbleibende Oberflächentherapeutika vorgesehen. Bei intraokularen Tamponaden (Endotamponaden) auf Silikonölbasis handelt es sich sogar um dauernd verbleibende oder nach geraumer Zeit zu entfernende Mittel.

Öle werden in der Medizin generell dann verwendet, wenn eine hohe Grenzflächenspannung und eine schwache Interaktion mit den wässrigen Systemen des Körpers angestrebt werden. Öle werden erst unter Zugabe von Emulgatoren oder spezifischen Enzymsystemen abgebaut. Ein natürlicher Abbau von Silikonöl ist nicht möglich, da der Körper über kein geeignetes Enzymsystem zur Aufspaltung der Moleküle verfügt. Niedermolekulare Anteile des Silikonöls werden aber verdächtigt, Ursache von gewissen degenerativen Prozessen im Körper zu sein. So werden ihnen zum Beispiel bei Verwendung als Glaskörperersatzstoff Beschädigungen des neuronalen Apparates der Netzhaut zugeschrieben (Saitoh et al., Long-term effect on optic nerve of silicone oil tamponade in rabbits: histological and EDXA findings. Eye, 2002, 16(2):171-176; Agrawal et al., Silicone oil-associated optic nerve degeneration. Am. J. Ophthalmol., 2002, 133(3):429 - 430).

J. Nepp et al. (Biomaterials 22 (2001) 3305-3310) beschreiben die klinische Verwendung von viscoelastischen künstlichen Tränen auf Basis von Natriumhyaluronat alleine oder in Verbindung mit Chondoitinsulfat zur Behandlcung von Augentrockenheit.

Andere biokompatible Substanzen mit hohen Oberflächenspannungen und geringer Neigung zur Schaumbildung (Emulsifikation) sind daher grundsätzlich interessante Kandidaten für den Ersatz von Glaskörper, Tränen und zur nicht wässrigen Behandlung von Haut und Schleimhäuten.

Der Anmelder hat im Rahmen seiner Forschungsarbeit festgestellt, dass eine bestimmte, als solche bereits bekannte, ölartige aber nicht silikonölartige Substanz sich hervorragend als Körperersatzstoff eignet und ohne jegliche Nachteile zum Beispiel als künstliche Tränensubstanz oder als künstlicher Glaskörper benutzt werden kann.

Diese als solche bereits bekannte und auf dem Markt befindliche Substanz ist Dimerdiol. Unter Dimerdiolen werden in der gegenwärtigen Anmeldung überwiegend aliphatische oder cycloaliphatische zweiwertige Alkohole enthaltende Produkte verstanden, die generell durch Dimerisation von ungesättigten Fettsäuren und nachfolgender Reduktion der Säuregruppen zu Hydroxylgruppen gewonnen werden. Solche Dimerdiole finden seit Jahrzehnten weitverbreitet Verwendung beispielsweise als Monomeren zur Herstellung von Kunststoffen, insbesondere von Polyurethanen (siehe zum Beispiel DE 43 08 100, WO 95/34592).

Dimerdiole sind ebenfalls bereits bekannt in kosmetischen und pharmazeutischen Zusammensetzungen, wo sie allerdings nur in geringen, nicht über ungefähr zwanzig Prozent hinausgehenden Anteilen verwendet werden (siehe zum Beispiel EP 1 471 898, DE 195 07 203, US 2001/0018424 und WO 98/47366).

Nach bestem Wissen des Anmelders sind Dimerdiole in relativ reiner Form allerdings bisher noch nie für eine medizinische Verwendung in Erwägung gezogen worden. Der Anmelder hat Dimerdiole auf ihre Biokompatibilität getestet und hat gefunden, dass diese Stoffe eine hervorragende Verträglichkeit auf der Augenoberfläche wie auch im Kontakt mit der Nervenfaserschicht des Auginneren aufweisen. Dimerdiol kann daher vorteilhaft die bisher verwendeten Silikonöle ersetzen.

Dimerdiole sind klar durchsichtig und vermischen sich nicht mit Wasser. Sie sind geeignet, auf der Augenoberfläche und im Auginneren wie auch an jeder anderen Schleimhaut und Körperhöhle eine Lipidschicht hoher Oberflächenspannung herzustellen. Damit eignen sich diese Stoffe hervorragend dazu, empfindliche Oberflächen durch die hohe Oberflächenspannung zu stabilisieren und zu tamponieren, wie dies bei einer inneren Schienung der Netzhaut durch einen Glaskörperersatzstoff notwendig ist.

Ein erster Gegenstand der Erfindung ist daher eine mindestens 90 Gewichtprozent, bevorzugterweise mindestens 95 Gewichtsprozent, und idealerweise mindestens 98 Gewichtsprozent Dimerdiol enthaltende Zusammensetzung zur Anwendung in einem Verfahren zur chirurgischen oder therapeutischen Behandlung des menschlichen oder tierischen Körpers gemäß unabhängigem Anspruch 1..

Wie bereits eingangs erklärt, sind diese Zusammensetzungen als solche nicht neu, da Dimerdiole in fast purer Form bereits in nicht medizinischen Bereichen verwendet worden sind.

Pharmazeutische Stoffgemische die einen hohen Anteil, d.h. einen Anteil von mindestens 90 Gewichtsprozent, an Dimerdiolen aufweisen sind allerdings nach bestem Wissen der Anmelderin noch nicht bekannt und die gegenwärtige Erfindung hat daher ebenfalls zum Gegenstand eine sterile und steril verpackte, mindestens 90 Gewichtprozent Dimerdiol enthaltende Zusammensetzung, welche außerdem mindestens einen pharmazeutischen Wirkstoff enthält.

Im Rahmen dieser therapeutischen oder chirurgischen Behandlung, werden die Zusammensetzungen der Erfindung als Körperflüssigkeitsersatzstoff ausgewählt aus der Gruppe bestehend aus künstlichen Tränen, künstlichem Schleim und Endotamponaden, verwendet.

Die in den erfindungsgemässen Stoffgemischen enthaltenen pharmazeutischen Wirkstoffe sind bevorzugt ausgewählt ist aus der Gruppe bestehend aus fettlöslichen Vitaminen, Steroiden, Antibiotika und immunmodulatorischen Wirkstoffen. Diese pharmazeutischen Wirkstoffe sind vorteilhafterweise in den Dimerdiol-zusammensetzungen löslich und gelöst, besonders in den Anwendungen wo es auf eine gute Transparenz ankommt. Die erfindungsgemässen Zusammensetzungen enthalten vorteilhafterweise insgesamt ungefähr 0,01 % bis 1 %, bevorzugsweise zwischen 0,1 bis 0,5 %, mindestens eines pharmazeutischen Wirkstoffe.

Die Dimerdiolzusammensetzungen können auf die Augenoberfläche aufgetragen oder in den Tränensack eingeführt werden, zum Beispiel zur Behandlung von Augentrockenheit, von Zuständen nach Verbrennung und Verätzung des Auges, von Lyell-Syndrom, von *Pemphigus vulgaris,* von okulärem Pemphigoid, von Hornhautentzündungen, von Hornhautulzera und von Hornhauterosionen.

Sie können ebenfalls auf die Nasenschleimhaut aufgetragen werden zur Behandlung von Nasenschleimhautaffektionen und -entzündungen.

Im Rahmen der Verwendung als Endotamponade, sind die Dimerdiolzusammensetzungen sowohl in Körperhöhlen mit Epithelauskleidung als auch in Körperhöhlen ohne Epithelauskleidung nützlich.

Der Glaskörperraum ist eine Körperhöhle ohne Epithelauskleidung und die Dimerdiolzusammensetzungen können dort zum Beispiel als Entotamponade zur Behandlung von Netzhautablösung, von proliferativer Vitreoretinopathie, von Glaukomen, von Uveitis, von Sehnervenentzündung und zur langfristigen Behandlung entzündlicher Netzhauterkrankungen verwendet werden.

Als Endotamponade von mit Epithelauskleidung versehenen Körperhöhlen seien zum Beispiel Gallenblasentamponaden, Blasentamponaden und Darmtamponaden genannt, welche zur Behandlung von entzündlichen oder postoperativen Reizzuständen der entsprechenden Körperhöhlen dienen.

Wie bereits eingangs erwähnt, sind die erfindungsgemäß verwendeten Dimerdiole, auch Dimeralkohole genannt, Mischungen welche überwiegend aliphatische oder cycloaliphatische zweiwertige Alkohole enthalten und durch Reduktion von Dimerfettsäuren erhalten werden können. Dimerfettsäuren ihrerseits werden durch Dimerisation ungesättigter Fettsäuren hergestellt.

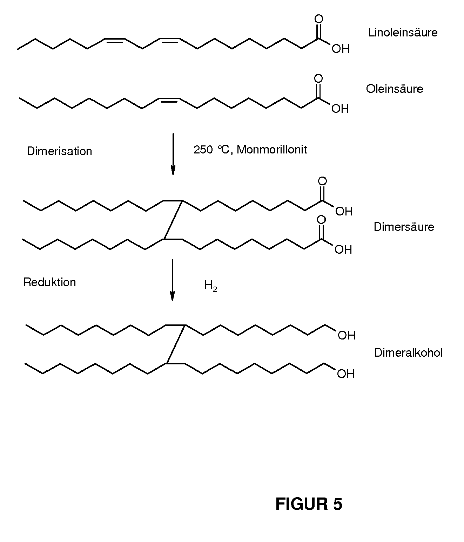

Das in Figur 5 dargestellte, stark vereinfachte Schema (Karlheinz Hill, Pure Appl. Chem., Vol. 72, No. 7, Seite 1255 - 1264, 2000) zeigt die von Linoleinsäure und Oleinsäure ausgehende Synthese eines C36-Dimerdiols.

Dimeralkohole enthalten generell einen gewissen synthesebedingten Anteil an Monomeren und Trimeren. Dieser Anteil ist in den erfindungsgemäßen Zusammensetzungen insgesamt geringer als 10 %, bevorzugterweise geringer als 8 %, besonders bevorzugt geringer als 5 %, und idealerweise geringer als 3 %, bezogen auf die Summe von Monomeren, Dimeren (Dimerdiolen) und Trimeren. Diese Prozentangaben sind, wie alle anderen Prozentangaben dieser Anmeldung, als Gewichtsprozente zu verstehen.

Wie bereits eingangs erwähnt, müssen die erfindungsgemäß verwendeten Dimerdiole selbstverständlich zur beabsichtigten Verwendung als Körperflüssigkeitsersatzstoffe, sowohl steril sein als auch steril verpackt werden. Die Sterilisation kann in einer dem Fachmann bekannten Weise durchgeführt werden, zum Beispiel durch Sterilfiltration, Bestrahlung mit Gammastrahlen oder Erhitzen, beispielsweise ungefähr eine Stunde bei 130 °C.

Die sterile Verpackung kann ebenfalls in dem Fachmann bekannter Weise erfolgen. Geeignete Behältnisse sind zum Beispiel Fläschchen, insbesondere Augentropfenfläschchen, Ampullen, Fertigspritzen oder Beutel.

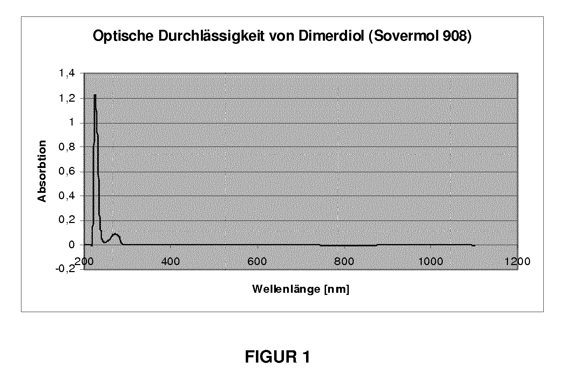

Die erfindungsgemäß verwendeten Dimerdiole und die erfindungsgemäßen pharmazeutischen Zusammensetzungen sollten so durchsichtig wie möglich sein, insbesondere wenn sie als Glaskörperersatzstoff verwendet werden. Die Figur 1 zeigt das Absorptionsspektrum eines für die Erfindung gut verwendbaren Dimerdiols (Sovermol 908, Cognis Deutschland GmbH). Man kann feststellen, dass dieses Produkt im gesamten sichtbaren und infraroten Bereich völlig durchsichtig ist.

Die erfindungsgemäß verwendeten Dimerdiole dürfen, insbesondere in der Applikation als Glaskörperersatzstoff, nicht zu dünnflüssig sein, da sie sonst dem Auge nicht den nötigen Halt bieten. Andererseits sollte ihre Viskosität nicht so hoch sein, dass ihre Tropffähigkeit oder Spritzfähigkeit beeinträchtigt werden könnte.

Die Dimerdiole haben eine nach ISO 2555 mit einem Rotationsviskosimeter Rheotec RC02 bei 25 °C bestimmte Brookfieldviskosität von ungefähr 1500 - 3200 mPa.s, besonders bevorzugt von ungefähr 1800 - 2800 mPa.s.

Wie bereits eingangs erwähnt, bieten Dimerdiole den Vorteil einer relativ hohen Oberflächenspannung. Diese beträgt generell mindestens 30 mN/m (gemessen nach der Wilhelmy-Plate Methode), vorzugsweise mindestens 35 mN/m. Diese hohe Oberflächenspannung ist äußerst wichtig, wenn die Dimerdiole als Endotamponade, insbesondere als Glaskörperersatzstoff verwendet werden. Sie garantiert nämlich, dass die Dimerdiolzusammensetzung nicht im Laufe der Zeit mit der wässrigen Körperflüssigkeit aus der direkten Umgebung des Glaskörpers emulgiert, was unausweichlich zu einer inakzeptablen Trübung führen würde.

Außer den vorgehend genannten Eigenschaften (Durchsichtigkeit, Viskosität, Oberflächenspannung) müssen die erfindungsgemäß verwandten Dimerdiole natürlich völlig biokompatibel und dürfen nicht zytotoxisch sein.

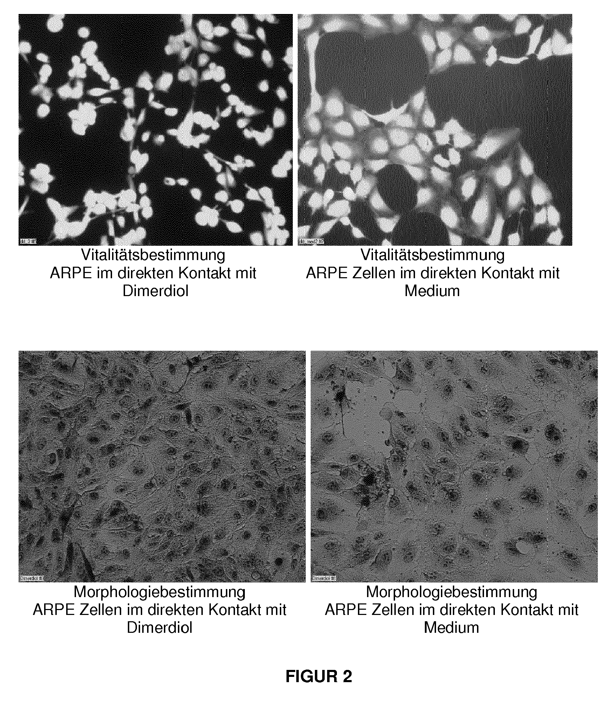

Der Anmelder hat adulte retinale Pigment-Epithelzellen (ARPE Zellen) 24 Stunden lang in direktem Kontakt mit einem auf dem Markt befindlichen und erfindungsgemäss bevorzugten Dimerdiol (Sovermol 908, Cognis Deutschland GmbH) in Kultur gesetzt und anschließend eine Vitalitäts- und Morphologiebestimmung im Vergleich mit einer Kultur auf einem üblichen Kulturmedium (MEM und Serum FCS 10 %) durchgeführt. Die Figur 2 zeigt die Ergebnisse dieser Versuche. Mann kann erkennen, dass die Epithelialzellen in keiner Weise unter dem Austausch des üblichen Kulturmediums durch den Dimerdiol leiden. Weder die Vitalität noch die Morphologie der Zellen nach 24 Stunden auf Dimerdiol ist beeinträchtigt.

Diese verschiedenen Untersuchungen zeigen somit, dass Dimerdiole hervorragende Kandidaten als Körperflüssigkeitsersatzstoffe sind.

In einer anderen Versuchsreihe hat der Anmelder im Tierversuch den Einfluss von Dimerdiol auf den Heilungsprozess der Hornhaut getestet. Als positive Kontrolle sind dabei die Augentropfen HYLO-COMOD^{®} (sterile Hyaluronsäure und Citratpuffer enthaltende wässrige Lösung) verwendet worden. Das nachfolgende Beispiel enthält eine ausführliche Beschreibung dieser Versuche, die gezeigt haben, dass Dimerdiol eine der positiven Kontrollsubstanz vergleichbare gute Heilwirkung der Kaninchenhornhaut und keinerlei Toxizität aufweist und somit ein vielversprechender Kandidat als Augentropfen oder Tränenersatzstoff ist.

### Beispiel

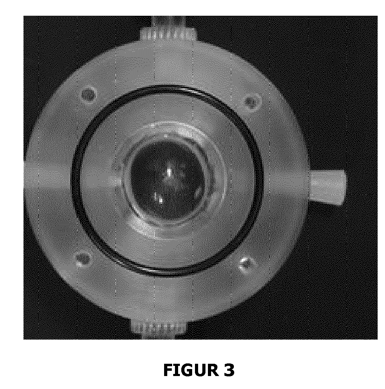

Im *Ex Vivo Eye Irritation Test* (EVEIT) (Figur 3) können kultivierte Hornhäute über Tage außerhalb des Tieres am Leben erhalten. Kleine Oberflächenveränderungen heilen in diesem System selbstständig zu. Die Hornhäute werden dazu durchgehend (6 µJ pro Minute) mit MEM Nährmedium *(Minimal essential medium* ohne Kalbserum) versorgt.

Nach einer Stabilisationszeit von zwölf Stunden werden für jeden Test auf je fünf Kaninchenhornhäuten kleine Erosionen aufgebracht. Unter Fluoresceinfärbung im blauen Licht leuchten diese Verletzungen gelb-grün und deren Heilung kann daher gut unter der Lupe verfolgt werden.

Auf jede Hornhaut werden während des Tages 12 Mal täglich mit einer zentralen Kanüle je 200 µl Hylo Comod^{®} Augentropfen (Kontrolle) oder je 200 µl Dimerdiol (Erfindung) im Zentrum der Hornhaut aufgebracht. In der Nacht werden die Hornhäute kontinuierlich mit MEM versorgt.

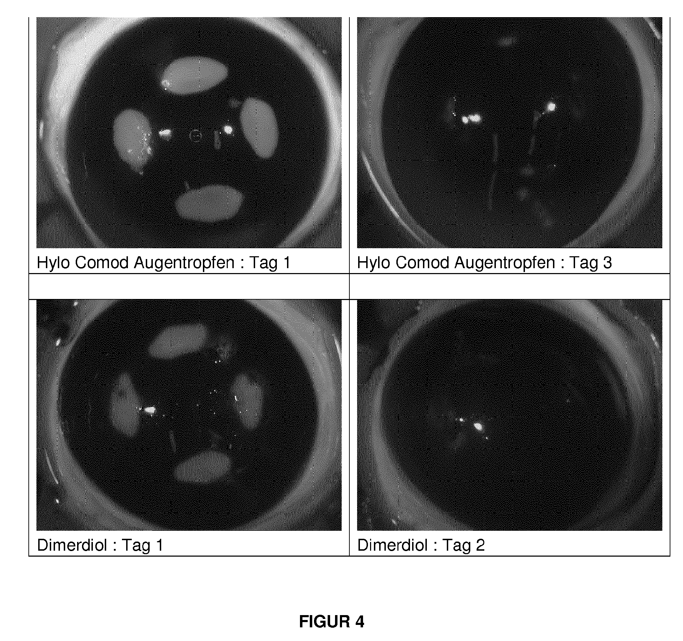

In nur drei Tagen erfolgt fast völlige Heilung mit dem bekannten Tränenersatzstoff Hylo Comod^{®} Augentropfen: gelb-grüne Flächen werden kleiner und verschwinden fast völlig. Noch besser heilt die Oberfläche allerdings mit dem erfindungsgemässen Dimerdiol. Nach nur zwei Tagen ist keine Verletzung mehr zu erkennen (Figur 4)

Desweiteren ist offenbart:
1. Zusammensetzung enthaltend mindestens 90 Gewichtprozent Dimerdiol zur Anwendung in einem Verfahren zur chirurgischen oder therapeutischen Behandlung des menschlichen oder tierischen Körpers.
2. Zusammensetzung nach 1, zur Anwendung als Körperflüssigkeitsersatzstoff ausgewählt aus der Gruppe bestehend aus künstlichen Tränen, künstlichem Schleim und Endotamponaden.
3. Zusammensetzung nach 1 oder 2, zur Anwendung in einem Verfahren zur Behandlung von Schleimhauttrockenheit oder Augentrockenheit.
4. Zusammensetzung nach 1 oder 2, zur Anwendung als Endotamponade in Körperhöhlen ohne Epithelauskleidung.
5. Zusammensetzung nach 1 oder 2, zur Anwendung als Endotamponade in Körperhöhlen mit Epithelauskleidung.
6. Zusammensetzung nach 1 bis 5, dadurch gekennzeichnet, dass der Gewichtsanteil von Dimerdiol mindestens 95 Gewichtsprozent, bevorzugt mindestens 98 Gewichtsprozent beträgt.
7. Zusammensetzung nach 1 bis 6, dadurch gekennzeichnet, dass sie eine Brookfieldviskosität bei 25 °C (nach ISO 2555) von 1500 - 3200 mPa.s, vorzugsweise von 1800 - 2800 mPa.s aufweist.
8. Zusammensetzung nach 1 bis 7, dadurch gekennzeichnet, dass sie steril und von der Umgebung dicht abgeschlossen in einem Behältnis verpackt ist.
9. Zusammensetzung nach 1 bis 8, dadurch gekennzeichnet, dass sie außerdem mindestens einen pharmazeutischen Wirkstoff enthält, bevorzugt ausgewählt aus der Gruppe bestehend aus fettlöslichen Vitaminen, Steroiden, Antibiotika und immunmodulatorischen Wirkstoffen.
10. Zusammensetzung enthaltend mindestens 90 Gewichtprozent Dimerdiol, dadurch gekennzeichnet, dass sie steril und steril verpackt ist und mindestens einen pharmazeutischen Wirkstoff enthält.
11. Zusammensetzung nach 10, dadurch gekennzeichnet, dass der pharmazeutische Wirkstoff ausgewählt ist aus der Gruppe bestehend aus fettlöslichen Vitaminen, Steroiden, Antibiotika und immunmodulatorischen Wirkstoffen.
12. Zusammensetzung nach 10 oder 11, dadurch gekennzeichnet, dass der Gewichtsanteil von Dimerdiol mindestens 95 Gewichtsprozent, bevorzugt mindestens 98 % beträgt.
13. Zusammensetzung nach 10 bis 12, dadurch gekennzeichnet, dass sie eine Brookfieldviskosität bei 25 °C (nach ISO 2555) von 1500 - 3200 mPa.s, vorzugsweise von 1800 - 2800 mPa.s aufweist.

## Patentansprüche

1. Zusammensetzung enthaltend mindestens 90 Gewichtprozent Dimerdiol enthaltend eine Mischung aus Monomeren, Dimeren und Trimeren, zur Anwendung in einem Verfahren zur chirurgischen oder therapeutischen Behandlung des menschlichen oder tierischen Körpers als Körperflüssigkeitsersatzstoff ausgewählt aus der Gruppe bestehend aus künstlichen Tränen, künstlichem Schleim und Endotamponaden, wobei das Dimerdiol weniger als 10 Gewichtsprozent Monomere und Trimere bezogen auf die Summe der Monomeren, Dimeren und Trimeren enthält, eine Brookfieldviskosität nach ISO 2555 bei 25°C von 1500 - 3200 mPa.s, vorzugsweise von 1800 - 2800 mPa.s und eine Oberflächenspannung von mindestens 30mN/m, gemessen nach der Wilhelmy-Plate Methode, aufweist.

2. Zusammensetzung zur Anwendung nach Anspruch 1, zur Anwendung in einem Verfahren zur Behandlung von Schleimhauttrockenheit oder Augentrockenheit.

3. Zusammensetzung zur Anwendung nach Anspruch 1, zur Anwendung als Endotamponade in Körperhöhlen ohne Epithelauskleidung.

4. Zusammensetzung zur Anwendung nach Anspruch 1, zur Anwendung als Endotamponade in Körperhöhlen mit Epithelauskleidung.

5. Zusammensetzung zur Anwendung nach Anspruch 1 bis 4, **dadurch gekennzeichnet, dass** der Gewichtsanteil von Dimerdiol mindestens 95 Gewichtsprozent, bevorzugt mindestens 98 Gewichtsprozent beträgt.

6. Zusammensetzung zur Anwendung nach einem der Ansprüche 1 bis 5, **dadurch gekennzeichnet, dass** sie eine Brookfieldviskosität nach ISO 2555 bei 25°C von 1800 - 2800 mPa.s aufweist.

7. Zusammensetzung zur Anwendung nach einem der Ansprüche 1 bis 6, **dadurch gekennzeichnet, dass** sie steril und von der Umgebung dicht abgeschlossen in einem Behältnis verpackt ist.

8. Zusammensetzung zur Anwendung nach einem der Ansprüche 1 bis 7, **dadurch gekennzeichnet, dass** sie außerdem mindestens einen pharmazeutischen Wirkstoff enthält, bevorzugt ausgewählt aus der Gruppe bestehend aus fettlöslichen Vitaminen, Steroiden, Antibiotika und immunmodulatorischen Wirkstoffen.

## Claims

1. A composition containing at least 90% by weight of dimer diol containing a mixture of monomers, dimers and trimers, for use in a method for the surgical or therapeutic treatment of the human or animal body as a body fluid replacement substance selected from the group comprising artificial tears, artificial mucous membrane and endotamponades, wherein the dimer diol contains less than 10% by weight of monomers and trimers with respect to the sum of monomers, dimers and trimers, has a Brookfield viscosity according to ISO 2555 at 25°C of from 1500 to 3200 mPa.s, preferably of from 1800 to 2800 mPa.s, and a surface tension of at least 30 mN/m, measured in accordance with the Wilhelmy-Plate method.

2. The composition according to Claim 1 for use in a method of treating dryness of the mucous membrane or xerophthalmia.

3. The composition according to Claim 1 for use as an endotamponade in body cavities without an epithelial lining.

4. The composition according to Claim 1 for use as an endotamponade in body cavities with an epithelial lining.

5. The composition according to Claims 1 to 4, **characterized in that** the proportion by weight of dimer diol amounts to at least 95% by weight, preferably at least 98% by weight.

6. The composition according to Claims 1 to 5, **characterized in that** it has a Brookfield viscosity according to ISO 2555 at 25°C of from 1800 to 2800 mPa.s.

7. The composition according to any one of Claims 1 to 6, **characterized in that** it is sterile and is packed - sealed off tightly from the environment in a container.

8. The composition according to any one of Claims 1 to 8, **characterized in that** it additionally contains at least one pharmaceutical active substance, preferably selected from the group comprising fat-soluble vitamins, steroids, antibiotics and immunomodulatory active substances.

## Revendications

1. Composition contenant au moins 90 % en poids de dimerdiol contenant un mélange de monomères, dimères et trimères pour l'utilisation dans un procédé de traitement chirurgical ou thérapeutique du corps humain ou animal en tant que substitut de liquides corporels choisis dans le groupe constitué de larmes artificielles, de mucus artificiel et d'endotamponnements, dans laquelle le dimerdiol contient moins de 10 % en poids de monomères et de trimères par rapport au total des monomères, dimères et trimères, présente une viscosité de Brookfield selon la norme ISO 2555 à 25 °C de 1500 à 3200 mPa.s, de préférence de 1800 à 2800 mPas.s et une tension de surface d'au moins 30 mN/m, mesurée selon le procédé de Wilhelmy-Plate.

2. Composition pour son utilisation selon la revendication 1, pour son utilisation dans un procédé de traitement de la sécheresse des muqueuses ou de la sécheresse oculaire.

3. Composition pour son utilisation selon la revendication 1, pour son utilisation en tant qu'endotamponnement dans des cavités corporelles sans revêtement endothélial.

4. Composition pour son utilisation selon la revendication 1, pour son utilisation en tant qu'endotamponnement dans des cavités corporelles ayant un revêtement épithélial.

5. Composition pour son utilisation selon la revendication 1 à 4, **caractérisée en ce que** la proportion en poids de dimerdiol est au moins de 95 % en poids, de préférence d'au moins 98 % en poids.

6. Composition pour son utilisation selon l'une des revendications 1 à 5, **caractérisée en ce qu'**elle présente une viscosité de Brookfield selon la norme ISO 2555 à 25 °C de 1800 à 2800 mPa.s.

7. Composition pour son utilisation selon l'une des revendications 1 à 6, **caractérisée en ce qu'**elle est stérile et emballée hermétiquement fermée à l'environnement dans un contenant.

8. Composition pour son utilisation selon l'une des revendications 1 à 7, **caractérisée en ce qu'**elle contient en outre au moins une substance active pharmaceutique, de préférence choisie dans le groupe constitué de vitamines liposolubles, de stéroïdes, d'antibiotiques et de substances actives immunomodulatrices.
